# EUROPEAN PATENT APPLICATION

(11) **EP 4 696 360 A1**
(43) Date of publication of application: **18.02.2026**
(21) Application number: 23934962.4
(22) Date of filing: 30.10.2023
(51) Int. Cl.: A61M 25/10

(54) **BALLOON CATHETER**

(30) Priority: 23.04.2023 CN 202310443743
(71) Applicant: Shanghai MicroPort Medical (Group) Co., Ltd., Shanghai 201203 (CN)
(72) Inventor: HU, Meng, Shanghai 201203 (CN); ZHOU, Qi, Shanghai 201203 (CN); CAO, Chunhong, Shanghai 201203 (CN); WANG, Jing, Shanghai 201203 (CN); ZHANG, Zhenghai, Shanghai 201203 (CN)
(74) Representative: Marks & Clerk LLP
(86) International application number: PCT/CN2023/127570
(87) International publication number: WO 2024/221794

(57) **Abstract**

The present invention provides a balloon catheter including a balloon assembly and a catheter assembly. The balloon assembly includes a balloon body including a plurality of interconnected sub-balloons, each of the sub-balloons is a hollow annular structure; and all the sub-balloons are coaxially arranged so that inner surfaces of all the sub-balloons together define a fluid flow channel extending through the balloon body along an axis thereof. A distal end of the catheter assembly is inserted within the fluid flow channel and connected to at least a distal-most one of the sub-balloons. The catheter assembly defines a fluid inlet channel in communication with all the sub-balloons. The fluid inlet channel is configured for flow of an inflation medium therethrough into all the sub-balloons, thereby inflating the balloon body. During use of the balloon catheter in a blood vessel, blood flow therein will not be blocked, preventing ischemia of downstream tissues that may otherwise occur. Thus, the balloon is allowed to be deployed in an expanded configuration for a period of time long enough to ensure adequate absorption of a drug on an outer surface of the balloon assembly by the wall of the blood vessel, resulting in improved therapeutic outcomes.

## Description

### TECHNICAL FIELD

The present invention pertains to the field of medical devices, and more particularly relates to a balloon catheter.

### BACKGROUND

Balloon angioplasty was originally used to treat peripheral arterial disease, and percutaneous transluminal coronary angioplasty (PTCA) that emerged at a later time has offered a new option for the treatment of coronary artery disease. Bare-metal stents, despite their success in addressing to some extent potential complications of balloon dilation, including vessel wall dissection and acute vascular occlusion, are prone to the development of in-stent restenosis (ISR). The demand for reducing the incidence of ISR has given rise to drug-eluting stents (DESs). When deployed in a diseased blood vessel, a DES can continually release an immunosuppressive or anti-tumor drug over a prolonged period of time, effectively excessive neointimal growth of the blood vessel. However, this inhibition of intimal hyperplasia may lead to delayed endothelial healing. Further, DES implantation requires prolonged dual antiplatelet therapy, significantly increasing the risk of post-implantation bleeding.

A drug-coated balloon (DCB) can be obtained by applying a drug coating to the surface of a balloon. After a DCB is delivered into a blood vessel, it can be inflated into contact with the wall of the blood vessel. Consequently, the drug coating will separate from the balloon surface and instead adhere to the blood vessel wall, thus delivering its intended therapeutic effect on a lesion site in the blood vessel. DCBs offer many advantages as follows:
1) A drug can be brought into direct contact with a vessel wall and thereby uniformly delivered to a damaged area of the wall.
2) At an early stage of treatment with the most pronounced progression of neointimal growth, a high drug concentration can be obtained on a vessel wall, enabling effective inhibition of intimal hyperplasia. At a late stage of treatment, a much lower drug concentration can be obtained on a vessel wall, which is in favor of endothelialization and associated with a reduced risk of thrombosis.
3) Drug release can be accomplished without reliance on a polymer matrix, reducing the risk of inflammation.
4) The associated procedures are simpler and require shorter radiation exposure and reduced use of contrast media, providing increased surgical safety.
5) They exhibit excellent crossability through small vessel lesions, tortuous lesions and bifurcation lesions and do not require the use of a metal scaffold. After being treated with a DCB, a blood vessel can largely restore the original anatomy, with its blood flow pattern being less affected. Compared with double-layer stents, the problem of a reduced vascular lumen is avoided during ISR treatment.
6) Implantation of foreign matter can be avoided, leaving the patient with a chance of receiving subsequent treatment if necessary.
7) They allow for shorter dual antiplatelet medication, reducing the incidence of bleeding complications and associated medical cost. Therefore, they are particularly suitable for patients who are intolerant to antiplatelet therapy or have recently received surgical treatment.

However, existing DCBs are still associated with some inherent drawbacks. For example, a DCB, when deployed in an expanded configuration in a blood vessel, will block blood flow in the vessel and therefore has to be deployed in this configured only for a limited period of time. This is because severe ischemia of distal tissues may be otherwise caused, which is detrimental to the patient's well-being. On the other hand, such short-term balloon dilation may disallow adequate absorption of the drug coating by the vessel wall. As a consequence, transfer of the drug may be too inefficient to enable satisfactory eventual therapeutic outcomes.

### SUMMARY

It is an object of the present invention to provide a balloon catheter, which can be expanded in a blood vessel without occluding it.

To this end, the present invention provides a balloon catheter including a balloon assembly and a catheter assembly.

The balloon assembly includes a balloon body, the balloon body including a plurality of interconnected sub-balloons, each of the sub-balloons being a hollow annular structure; and all the sub-balloons being coaxially arranged so that inner surfaces of all the sub-balloons together define a fluid flow channel extending through the balloon body along an axis of the balloon catheter.

An outer diameter of a distal end of the catheter assembly is smaller than a diameter of the fluid flow channel, the distal end of the catheter assembly inserted within the fluid flow channel, and the distal end of the catheter assembly is at least connected to a distal-most one of the sub-balloons, the catheter assembly further provided with a fluid inlet channel, the fluid inlet channel being in communication with all the sub-balloons.

Optionally, the balloon assembly may further include a covering membrane covering outer surfaces of at least some of the sub-balloons.

Optionally, the balloon catheter may further include a drug coating provided on an outer surface of the balloon assembly.

Optionally, the balloon body may be a helical structure formed by helically winding an elongate primary balloon around a central axis, wherein the sub-balloons are provided by respective helical turns of the helical structure and all the sub-balloons are in communication with each other.

Optionally, the primary balloon may include an elongate primary sub-balloon, all the helical turns are formed from the elongate primary sub-balloon.

Optionally, the primary balloon may include a plurality of primary sub-balloons, the plurality of primary sub-balloons are sequentially interconnected to form the primary balloon.

Optionally, each of the primary sub-balloons may be formed into at least one of the helical turns.

Optionally, a connection point may be formed between every two adjacent primary sub-balloons, and wherein all the connection points are aligned in a circumferential direction of the balloon body.

Optionally, the sub-balloons may be each in the shape of a circular ring, all the sub-balloons are not in communication with each other, and each of the sub-balloons is respectively connected to the fluid inlet channel.

Optionally, the balloon body may have an outer diameter in the range of 2 mm to 5 mm. Additionally or alternatively, the balloon body may have a length in the range of 10 mm to 40 mm along the axis of the balloon catheter. Additionally or alternatively, each sub-balloon may has a cross-section with a diameter in the range of 0.5 mm to 2 mm.

Optionally, the catheter assembly may include an inner tube and an outer tube, wherein the outer tube is disposed over a portion of an outer circumferential surface of the inner tube, an inner diameter of the outer tube being greater than an outer diameter of the inner tube, allowing the fluid inlet channel to be defined between an inner surface of the outer tube and the outer surface of the inner tube, a distal end of the inner tube being located external to a distal end of the outer tube, the distal end of the inner tube being inserted within the fluid flow channel and connected to at least the distal-most one of the sub-balloons.

Optionally, the balloon catheter may further include a radiopaque marker element provided at the distal end of the inner tube, which is configured to indicate a position of the balloon assembly.

The present invention offers the following advantages over the prior art:
It provides a balloon catheter including a balloon assembly and a catheter assembly. The balloon assembly includes a balloon body including a plurality of interconnected sub-balloons each in the shape of a hollow ring and all coaxially arranged so that their inner surfaces together define a fluid flow channel extending through the balloon body along an axis thereof. The catheter assembly has an outer diameter at a distal end thereof, which is less than a diameter of the fluid flow channel. The distal end of the catheter assembly is inserted within the fluid flow channel and connected to at least a distal-most one of the sub-balloons. The catheter assembly defines a fluid inlet channel in communication with all the sub-balloons. The fluid inlet channel is configured for flow of an inflation medium therethrough into all the sub-balloons, inflating the balloon body, or from the sub-balloons, deflating the balloon body. The balloon body may be provided with a drug coating on its outer surface. With this arrangement, when the balloon catheter is inserted into a diseased blood vessel and inflated therein, the drug coating on the balloon body can be brought into contact with, and absorbed by, a wall of the diseased blood vessel. At the same time, blood flow is still allowed in the blood vessel through the fluid flow channel. That is, during use of the balloon catheter in a blood vessel, blood flow therein will not be blocked, preventing ischemia of downstream tissues that may otherwise occur. Thus, the balloon is allowed to be deployed in an expanded configuration for a period of time long enough to ensure adequate absorption of a drug in the drug coating by the wall of the blood vessel, resulting in improved therapeutic outcomes.

### BRIEF DESCRIPTION OF THE DRAWINGS

The accompanying drawings are provided to facilitate a better understanding of the present invention and do not unduly limit the scope thereof in any sense, in which:
Fig. 1 shows a schematic partial view of a balloon catheter according to an embodiment of the present invention, showing a helical balloon body;
Fig. 2 shows a schematic partial view of a balloon catheter according to an embodiment of the present invention, showing a view in direction A of Fig. 1;
Fig. 3 schematically illustrates an application scenario of the balloon catheter of Fig. 1;
Fig. 4 shows a schematic partial view of a balloon catheter according to an embodiment of the present invention, showing a covering membrane;
Fig. 5 shows the balloon catheter of Fig. 4, as viewed along direction B;
Fig. 6 schematically illustrates an application scenario of the balloon catheter shown in Fig. 4;
Fig. 7 shows a schematic partial view of a balloon catheter according to an embodiment of the present invention;
Fig. 8 schematically illustrates an application scenario of the balloon catheter shown in Fig. 7; and
Fig. 9 schematically illustrates a partial structural schematic view of a balloon catheter according to an embodiment of the present invention, showing the structure of the proximal portion of the balloon catheter.

### List of Reference Numerals

100 balloon assembly; 110 balloon body; 111 sub-balloon; 101 fluid flow channel; 102 proximal connection section; 103 distal connection section; 120 covering membrane; 200 catheter assembly; 210 outer tube; 211 marker band; 220 inner tube; 230 proximal connector; 240 stress diffusion tube; 260 radiopaque marker element; 250 guide tip.

### DETAILED DESCRIPTION

Embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of" means "two or more", unless the context clearly dictates otherwise. As used herein, the term "or" is generally employed in the sense including "and/or", unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

As used herein, the terms "distal" and "proximal" may be used to describe positions and locations of various components and elements of a medical device generally relative to a patient. While not intended to be limiting, a "distal end" usually refers to an end that enters the patient's body first, while a "proximal end" usually refers to an end closer to an operator.

It is an object of the present invention to provide a balloon catheter having a distal portion intended to be deployed and expanded in a diseased blood vessel. In the expanded configuration, blood flow in the blood vessel will not be blocked, allowing the balloon catheter to be deployed for a prolonged period of time. As a result, a drug coating on the balloon catheter can be kept in contact with the wall of the blood vessel for a period of time long enough to allow adequate absorption of the drug by the vessel wall, promising improved therapeutic outcomes.

Objects, advantages and features of the present invention will become more apparent from the following detailed description of examples of embodiment thereof. Note that the figures are provided in a very simplified form not necessarily drawn to exact scale for the only purpose of helping to explain the disclosed embodiments in a more convenient and clearer way. Throughout these drawings, like numerals indicate like elements.

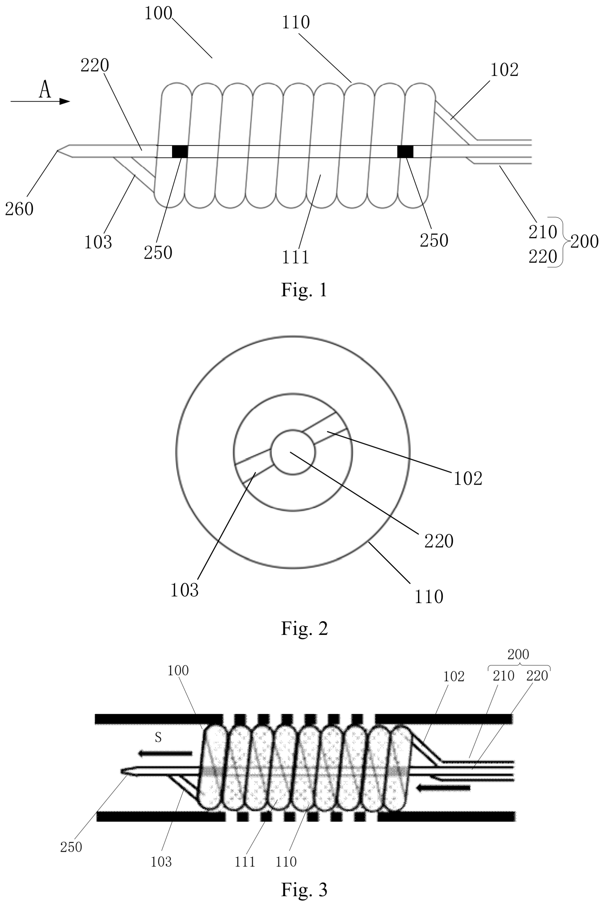

Figs. 1, 2, 4, 5 and 7 are schematic partial views of a balloon catheter according to embodiments of the present invention. As shown, the balloon catheter includes a balloon assembly 100 and a catheter assembly 200. The balloon assembly 100 includes a balloon body 110. The balloon body 110 includes a plurality of interconnected sub-balloons 111, each sub-balloon 111 is a hollow annular structure. All the sub-balloons 111 are coaxially arranged so that inner surfaces of the sub-balloons 111 are all coaxial and together define a fluid flow channel 101. The fluid flow channel 101 axially penetrates the balloon body 110 along the axis of the balloon catheter. The outer diameter of the distal end of the catheter assembly 200 is smaller than the diameter of the fluid flow channel 101. The distal end of the catheter assembly 200 is inserted within the fluid flow channel 101, and the distal end of the catheter assembly 200 is at least connected to the most distal sub-balloon 111. The catheter assembly 200 is further provided with a liquid injection channel (not labeled in the figures), which communicates with all the sub-balloons 111.

"The sub-balloon 111 is a hollow annular structure" means that the sub-balloon 111 has an inner cavity, and its projection on a plane perpendicular to the axis of the balloon body 110 extends 360° around the axis of the balloon body 110. In some embodiments, the annular structure is a closed circle. In alternative embodiments, each sub-balloon 111 has a first end and a second end. The first end and the second end are aligned in the circumferential direction of the balloon body 110 but offset from each other in the axial direction of the balloon body 110. Thus, each sub-balloon 111 forms an open-ended helical turn. According to embodiments of the present invention, the inner cavities of all the sub-balloons 111 together define an inflation chamber of the balloon body 110, which is isolated from the fluid flow channel. By "in communication with all the sub-balloons 111", it is intended to mean that the fluid inlet channel is in communication with the inflation chamber. With this arrangement, through the inflation chamber, an inflation medium can be introduced into the fluid inlet channel, causing radial expansion of the balloon body 110. Moreover, the inflation medium can be discharged from the inflation chamber, causing radial contraction of the balloon body 110. It will be understood that the radial expansion of the balloon body 110 is equivalent to radial expansion of the balloon assembly 100, and the radial contraction of the balloon body 110 is equivalent to radial contraction of the balloon assembly 100.

A drug coating (not shown) may be provided on an outer surface of the balloon assembly 100. Figs. 3, 6 and 8 schematically illustrate application scenarios of the balloon catheter. The balloon catheter can be used in interventional procedures such as transluminal coronary angioplasty.

The balloon assembly can be folded and crimped, the distal portion of the balloon catheter can be delivered into a diseased blood vessel. After that, an inflation medium may be introduced into the inflation chamber of the balloon body 110 through the fluid inlet channel, thereby inflating and radially expanding the balloon body 110 until the drug coating on the outer surface of the balloon assembly 100 is brought into contact with the wall of the diseased blood vessel, allowing the drug to be absorbed by the vessel wall. At the same time, blood flow in the blood vessel is still allowed through the fluid flow channel 101 in a direction as indicated by arrow S shown in Figs. 3, 6 and 9 toward distal tissues. In other words, the balloon assembly 100 in the balloon catheter proposed herein can be expanded in a blood vessel without blocking blood flow therein, preventing ischemia of downstream tissues that may otherwise occur. Accordingly, the balloon assembly 100 is allowed to be deployed in an expanded configuration for a period of time long enough to ensure sufficient contact of the drug coating with the blood vessel wall. Thus, the drug can be better absorbed by the blood vessel wall with increased utilization, resulting in improved therapeutic outcomes including better inhibition of intimal hyperplasia and less likelihood of restenosis of the blood vessel.

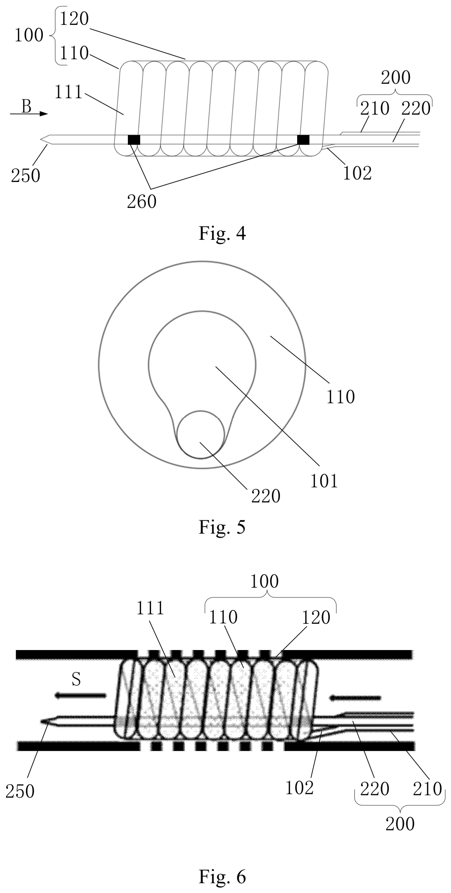

In some embodiments, the drug coating is directly provided on the balloon body 110, in particular on outer surfaces of at least some of the sub-balloons 111. In alternative embodiments, as shown in Figs. 4 and 5, the balloon assembly 100 further includes a covering membrane 120 covering the outer surfaces of at least some, preferably all of the sub-balloons. The covering membrane 120 has a uniform inner diameter and a uniform outer diameter across its entire axial length. The balloon body 110 may be radially expanded so that the covering membrane 120 is in the shape of a cylindrical tube. This shape enables contact of the balloon assembly 100 with the blood vessel wall over a larger area, and hence more adequate contact of the drug coating with the blood vessel wall. Those skilled in the art will understand that a projection of each sub-balloon 111 on a plane perpendicular to the axis of the balloon body 110 has an inner contour and an outer contour, which are both circular. The inner contour corresponds to the aforementioned "inner surfaces", and the outer contour corresponds to the aforementioned "outer surfaces".

Optionally, the drug coating may be formed on the outer surface of the balloon assembly 100 by various approaches including electrostatic spraying, atomized spraying, dip coating and crystal growth. In some embodiments, the drug coating consists of an active ingredient. In alternative embodiments, the drug coating contains an active ingredient and a carrier matrix. Examples of the active ingredient may include, but are not limited to, at least one of rapamycin, rapamycin derivatives, paclitaxel and paclitaxel derivatives. Examples of the carrier matrix may include, but are not limited to, at least one of iopromide, urea, lecithin and polylactic acid.

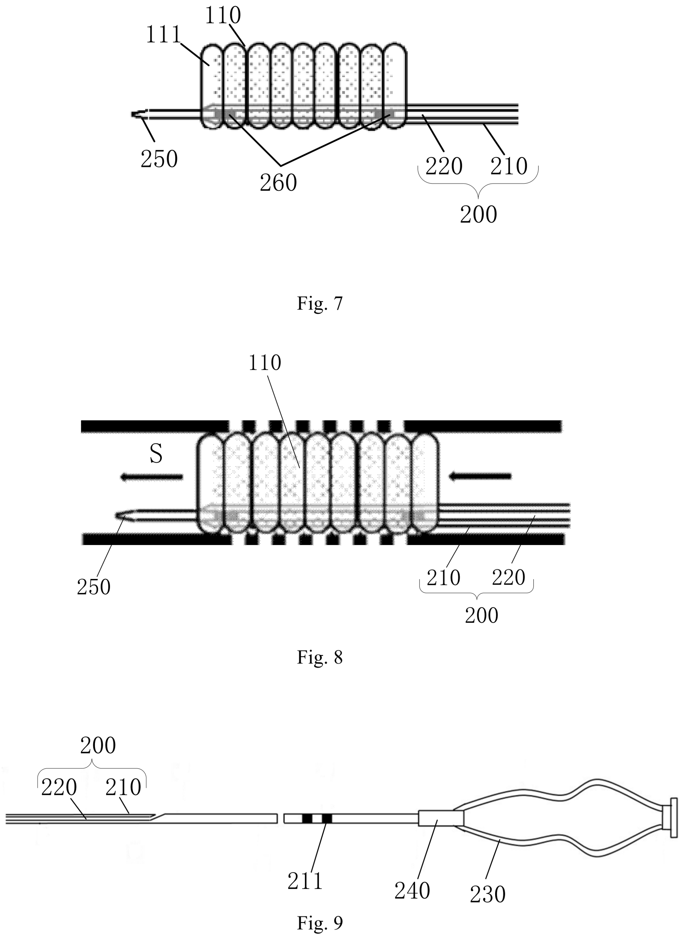

Further, as shown in Fig. 9, the catheter assembly 200 includes an outer tube 210 and an inner tube 220. The outer tube 210 is disposed over part of an outer circumferential surface of the inner tube 220, and the inner diameter of the outer tube 210 is greater than an outer diameter of the inner tube 220, allowing the fluid inlet channel to be defined between the inner surface of the outer tube 210 and the outer surface of the inner tube 220. A distal end of the inner tube 220 is located external to a distal end of the outer tube 210, and the inner tube 220 is sealingly connected to the distal end of the outer tube 210. The distal end of the inner tube 220 is inserted within the fluid flow channel and connected to at least the distal-most one of the sub-balloons 111.

The outer tube 210 may be made from a single continuous tube, or from two tubes connected to each other along the axis of the balloon catheter, without limiting the present invention. Preferably, the outer tube 210 is provided with a hydrophilic coating (not shown) over part of an outer circumferential surface thereof. The hydrophilic coating can lubricate a portion of the balloon catheter intended to be inserted into a patient's blood vessel, reducing the resistance that it encounters when moved in the blood vessel. In addition, the outer tube 210 is further provided around a proximal end thereof with a marker band 211 on its circumferential surface.

The balloon catheter further includes a proximal connector 230, which may be connected to the proximal end of the outer tube 210 via a stress diffusion tube 240. The proximal connector 230 communicates with the fluid inlet channel and is provided with a liquid injection interface (not shown). The liquid injection interface is configured for connection with a fluid source, from which the inflation medium can be introduced through the fluid inlet channel into the sub-balloons 111 to inflate the balloon body 110.

Optionally, the balloon catheter may further include a radiopaque marker element 260 provided on the outer surface of the inner tube 220 around the distal end thereof and configured to enable the position of the balloon assembly 100 to be visually identified. With this, during use of the balloon catheter, it is possible to determine whether the balloon assembly 100 has reached a target lesion site.

The catheter assembly 200 further includes a guide tip 250, which is attached to the distal end of the inner tube 220 and located on a distal side of the balloon body 110. Typically, the guide tip 250 is a conical structure whose outer diameter gradually decreases from the proximal end to the distal end. The guide tip 250 provides guidance for the advancement of the distal end of the balloon catheter within the vessel. Additionally, the catheter assembly defines a guide-wire lumen extending from a proximal end of the inner tube 220 to the guide tip 250 and penetrating through the guide tip 250. In an optional implementation, a first inlet (not shown) is provided in a side wall of the inner tube 220 around its proximal end so as to communicate with the guide-wire lumen, and a corresponding second inlet (not labeled) is provided in a side wall of the outer tube 210. A guide wire may be passed through the second and first inlets and thus partially received in the guide-wire lumen.

The structure and formation of the balloon body 110 and its connection with the catheter assembly 200 are set forth below.

In a non-limiting embodiment, the balloon body 110 is a helical structure obtained by helically winding an elongate primary balloon around a central axis so that each turn of the helical structure provides a respective one of the sub-balloons 111 (i.e., each sub-balloon 111 is in the form of a helical turn). In this embodiment, all the sub-balloons 111 are in mutual communication.

In one optional implementation, the primary balloon includes a single long primary sub-balloon, from which all the helical turns are formed. That is, the primary balloon is an integral one-piece structure. Accordingly, the formation of the helical balloon body 110 may include the steps described below.

At first, a tubular balloon material is blow-molded so that part of it forms the primary sub-balloon that provides the entire primary balloon. The primary sub-balloon is axially flanked at both ends by respective remaining portions of the tubular balloon material, which are respectively retained as a proximal connection section 102 and a distal connection section 103 of the balloon body 110 (see Fig. 1). The tubular balloon material may be any of polyether block amide (Pebax), thermoplastic polyurethane and thermoplastic polyester elastomer (TPEE). The primary balloon has an outer diameter of 0.5 mm to 2 mm, preferably 1 mm. The primary balloon has a length of 80 mm to 300 mm, preferably 100 mm.

Next, the primary sub-balloon is wound on a mandrel (not shown) and thereby shaped into the helical balloon body 110. It will be understood that the shape of the mandrel determines that of the balloon body 110. For example, if the mandrel is cylindrical, then the resulting balloon body 110 will be a cylindrical helical structure.

The size of the mandrel is chosen depending on predetermined dimensions of the balloon body 110, particularly on the diameter of the fluid flow channel 101. The diameter of the fluid flow channel may be equal to the difference between an outer diameter of the balloon body 110 and twice the outer diameter of the primary balloon. In some embodiments, the diameter of the fluid flow channel may range from 0.5 mm to 2 mm, and accordingly, the outer diameter of the mandrel may also be in the range of 0.5 mm to 2 mm. In one particular implementation, the diameter of the fluid flow channel may be controlled at 1 mm, and the mandrel may have an axial length not less than an axial length of the balloon body 110. In embodiments of the present invention, the axial length of the balloon body 110 may range from 10 mm to 40 mm. In addition, when the balloon body 110 is inflated, the sub-balloons 111 may each has a circular annular cross-section with an outer diameter equal to the outer diameter of the primary balloon, i.e., 0.5-2 mm. Further, each sub-balloon 111, when straightened, may have a length of 6 mm to 12 mm.

Subsequently, outer walls of each adjacent pair of helical turns (i.e., sub-balloons 111) are connected together by adhesive bonding or any other suitable method, preventing relaxation of the helical structure.

Afterwards, the mandrel is removed.

Forming the balloon body 110 in this way offers the advantage of fewer process steps. In this embodiment, the balloon body 110 and the catheter assembly 200 are assembled together so that the distal connection section 103 is connected to the inner tube 220 and that the proximal connection section 102 is connected to the outer tube 210 so as to communicate with the fluid inlet channel.

In an alternative embodiment, the elongate primary balloon includes multiple primary sub-balloons, which are separately formed and then connected together. This process may include the steps described below.

First of all, multiple tubular balloon materials are separately blow-molded into the individual primary sub-balloons each having first and second connection sections at its two respective axial ends, which are provided by respective retained portions of the corresponding tubular balloon material. Each primary sub-balloon may have a length of 6 mm to 10 mm, such as 10 mm.

After that, the second connection section of one primary sub-balloons is connected to the first connection section of another primary sub-balloon by welding or any other suitable method. This process is repeated until the primary sub-balloons are all interconnected end-to-end, forming the longer primary balloon. Accordingly, the first connection section of the primary sub-balloon at one axial end of the primary balloon provides the proximal connection section 102 of the balloon body 110, and the second connection section of the primary sub-balloon at the other axial end of the primary balloon provides the distal connection section 103 of the balloon body 110. It will be understood that since each adjacent pair of primary sub-balloons has a connection point between the two elements, there are multiple such connection points on the primary balloon.

Next, the primary balloon is wound on a mandrel (not shown) and thereby shaped into the helical balloon body 110. In this step, each primary sub-balloon may form at least one helical turn, and preferably, all the connection points are aligned circumferentially around the balloon body 110. This can facilitate subsequent crimping of the balloon body 110 to a minimum possible outer diameter of the balloon catheter. In one optional implementation, each primary sub-balloon may be shaped into a single helical turn, which provides a respective one of the sub-balloons 111. In alternative implementations, each primary sub-balloon may be shaped into two or more helical turns, e.g., two, three or another number of helical turns.

Afterwards, outer walls of each adjacent pair of helical turns (i.e., sub-balloons 111) are connected together by adhesive bonding or any other suitable method, preventing relaxation of the helical structure, followed by removal of the mandrel.

In this embodiment, the distal connection section 103 of the balloon body 110 is connected to the inner tube 220, and the proximal connection section 102 of the balloon body 110 is connected to the outer tube 210 so as to communicate with the fluid inlet channel. In this embodiment, despite a larger number of process steps involved in the formation of the balloon body 110, this process itself is simple and easy to implement and does not require the use of a complex mold. In addition, lumens of the sub-balloons 111 in the resulting balloon body 110 are in mutual communication. In other words, the entire balloon body 110 can be brought into communication with the fluid inlet channel simply by fluid-connecting the proximal connection section 102 to the channel. This can reduce the risk of leakage of the inflation medium.

In yet an alternative embodiment, as shown in Figs. 7 and 8, the sub-balloons 111 are in the shape of circular rings, which are separate from one another and in individual communication with the fluid inlet channel. In this embodiment, the formation of the balloon body 110 may include the steps described below.

First of all, multiple tubular balloon materials are separately blow-molded into respective primary sub-balloons each with portions of the corresponding tubular balloon material being retained at its respective axial ends as proximal and distal connection sections (not shown). Each primary sub-balloon may have a diameter of 0.5 mm to 2 mm, such as 1 mm, and a length of 6 mm to 10 mm.

Each of the primary sub-balloons is then formed into a ring, and its proximal and distal end connection sections are joined together by welding or any other suitable method, obtaining a respective one of the sub-balloons 111. After this step, each proximal connection section is kept open at its proximal end, allowing the balloon body 110 to be subsequently assembled with the catheter assembly 200 so that each proximal connection section is connected to the outer tube 210 so as to bring the respective sub-balloon 111 into communication with the fluid inlet channel.

After that, all the sub-balloons 111 are coaxially arranged and outer walls of each adjacent pair of sub-balloons 111 are connected together by adhesive bonding or any other suitable method.

In this embodiment, despite a larger number of process steps involved in the formation of the balloon body 110, this process itself is simple and easy to implement and does not require the use of a complex mold.

The balloon body 110 is assembled with the catheter assembly 200 so that the distal connection section of a distal-most one of the sub-balloons 111 is connected to the inner tube 220 and that the proximal connection sections of all the sub-balloons 111 are connected to the outer tube 210 so as to communicate with the fluid inlet channel. With this arrangement, during practical use of the balloon catheter, the inflation medium can flow through the fluid inlet channel and the individual proximal connection sections into the respective sub-balloons 111, dilating the sub-balloons 111. This design allows the catheter assembly 200 to be arranged alongside the periphery of the fluid flow channel and reduced in size, resulting in a larger flux area of the fluid flow channel, which allows more blood to flow therein.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A balloon catheter, comprising a balloon assembly and a catheter assembly,
the balloon assembly comprising a balloon body, the balloon body comprising a plurality of interconnected sub-balloons, each of the sub-balloons being a hollow annular structure; and all the sub-balloons being coaxially arranged so that inner surfaces of all the sub-balloons together define a fluid flow channel extending through the balloon body along an axis of the balloon catheter,
wherein an outer diameter of a distal end of the catheter assembly is smaller than a diameter of the fluid flow channel, the distal end of the catheter assembly inserted within the fluid flow channel, and the distal end of the catheter assembly is at least connected to a distal-most one of the sub-balloons, the catheter assembly further provided with a fluid inlet channel, the fluid inlet channel being in communication with all the sub-balloons.

2. The balloon catheter according to claim 1, wherein the balloon assembly further comprises a covering membrane, the covering membrane covering outer surfaces of at least some of the sub-balloons.

3. The balloon catheter according to claim 1 or 2, further comprising a drug coating, the drug coating being provided on an outer surface of the balloon assembly.

4. The balloon catheter according to claim 1 or 2, wherein the balloon body is a helical structure formed by helically winding an elongate primary balloon around a central axis, wherein the sub-balloons are provided by respective helical turns of the helical structure and all the sub-balloons are in communication with each other.

5. The balloon catheter according to claim 4, wherein the primary balloon comprises an elongate primary sub-balloon, all the helical turns are formed from the elongate primary sub-balloon.

6. The balloon catheter according to claim 4, wherein the primary balloon comprises a plurality of primary sub-balloons, the plurality of primary sub-balloons being sequentially interconnected to form the primary balloon.

7. The balloon catheter according to claim 6, wherein each of the primary sub-balloons is formed into at least one of the helical turns.

8. The balloon catheter according to claim 6, wherein a connection point is formed between every two adjacent primary sub-balloons, and wherein all the connection points are aligned in a circumferential direction of the balloon body.

9. The balloon catheter according to claim 1 or 2, wherein the sub-balloons are each in the shape of a circular ring, all the sub-balloons are not in communication with each other, and each of the sub-balloons is respectively connected to the fluid inlet channel.

10. The balloon catheter according to claim 1 or 2, wherein the balloon body has an outer diameter in the range of 2 mm to 5 mm, and/or the balloon body has a length in the range of 10 mm to 40 mm along the axis of the balloon catheter, and/or each sub-balloon has a cross-section with a diameter in the range of 0.5 mm to 2 mm.

11. The balloon catheter according to claim 1 or 2, wherein the catheter assembly comprises an inner tube and an outer tube, the outer tube disposed over a portion of an outer circumferential surface of the inner tube, an inner diameter of the outer tube being greater than an outer diameter of the inner tube, allowing the fluid inlet channel to be defined between an inner surface of the outer tube and the outer surface of the inner tube, a distal end of the inner tube being located external to a distal end of the outer tube, the distal end of the inner tube being inserted within the fluid flow channel and connected to at least the distal-most one of the sub-balloons.

12. The balloon catheter according to claim 11, further comprising a radiopaque marker element provided at the distal end of the inner tube, which is configured to indicate a position of the balloon assembly.
